# EUROPEAN PATENT APPLICATION

(11) **EP 1 127 557 A1**
(43) Date of publication of application: **29.08.2001**
(21) Application number: 00301498.2
(22) Date of filing: 25.02.2000
(51) Int. Cl.: A61F 2/06

(54) **Vascular graft**

(71) Applicant: EndoArt S.A., 1278 La Rippe (CH)
(72) Inventor: The designation of the inventor has not yet been filed
(74) Representative: Nicholls, Michael John

(57) **Abstract**

A vascular graft comprises a tubular vessel having a lumen. In cross-section, the lumen has a periphery which is non-circular and the orientation of the non-circular periphery rotates as a function of position along the length of the graft in order to impart rotational flow velocity components to a fluid passing along the graft to thereby alleviate the problem of stenosis, particularly at an end-to-side anastomosis.

## Description

This invention relates to a graft, for example for use as an artificial human blood vessel.

Vascular grafts are used as substitute vessels in humans and other animals in a variety of situations. Examples are bypass grafts, e.g. coronary artery bypass grafts, aorto-iliac bypass grafts, etc, connecting an artery to another artery, and arteriovenous shunts connecting an artery to a vein.

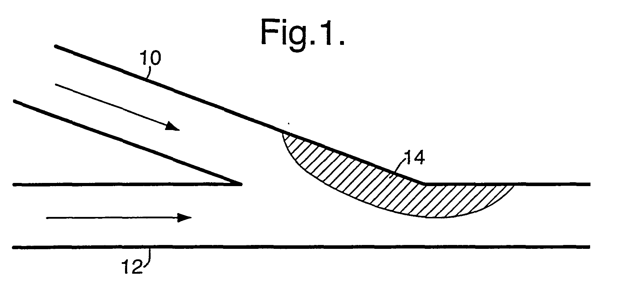

Each end of the grafted vessel (hereinafter referred to simply as the graft) is typically connected to an existing vessel in the body by an end-to-side anastomosis in which the end of the graft is sutured to an opening made in the side of the existing vessel. This is illustrated schematically in Fig. 1 where graft 10 is joined to vessel 12. However, a problem of stenosis or arterial disease can occur, especially in the vicinity of the end-to-side anastomosis at the distal end of the graft 10, the distal end being defined as the end of the graft at which the fluid flows out of the graft 10 into the vessel 12; the flow direction is indicated by the arrows in Fig. 1, and the stenosis is indicated at 14. The stenosis 14 is a narrowing of the vessel, and if unopened by angioplasty, the stenosis progresses until the vessel 12 is completely blocked. The stenosis is due to neo-intimal hyperplasia, which is the response of the vessel to abnormal flow conditions.

Various mechanisms are considered as possibly contributing to the stenosis development. The abrupt change in geometry at the end-to-side anastomosis results in large flow separation zones, turbulence and high wall shear stress gradients which have been observed and associated with stenosis, see, for example, Lei, M., Kleinstreuer, C., and Archie, J. P., *J Biomech Eng*, *119,* 343-8 (1997) and Ojha, M., *Circ Res, 74,* 1227-31 (1994). Other work has shown that, in nature, arterial bifurcations are non-planar which means that the mother and daughter branches of the bifurcation cannot be contained in the same plane. This geometry results in flow patterns which contain rotational velocity components in addition to axial velocity and this "swirling" flow has been found to make the distribution of shear stress in the vicinity of an anastomosis more uniform (Caro, C. G., *et al*, *Proc Roy Soc A 452,* 185-197 (1996)) as well as reducing the extent of flow separation and diminishing turbulence ( Doorly, D. J., *et al*, ASME BED Meeting, 1-8 (1997)).

Nevertheless, stenosis is a considerable problem, for example, in 90% of arteriovenous grafts (AV grafts), stenosis develops at the venous anastomosis side. AV graft survival is around only 1.5 years. Conventionally, alleviation of this problem requires surgery, such as angioplasty to remove the stenosis or surgery to implant a new AV graft in a different limb of the patient.

According to the present invention there is provided a graft comprising a tubular vessel having a lumen, wherein at least a portion of said lumen, in cross-section, has a non-circular periphery and wherein the orientation of said non-circular periphery rotates as a function of position along the length of said portion, for imparting rotational flow velocity components to a fluid passing along said graft.

In use, the graft according to the invention imparts rotational flow velocity components to a fluid, such as blood, passing along the graft. The fluid flowing out of the graft can thereby contain consistent rotational or secondary flows which tend to stabilise the overall flow. The flow at the anastomosis is thus smoother and more uniform with reduced turbulence and flow separation and more evenly distributed shear stress which alleviates unwanted tissue reaction, such as intimal hyperplasia in the vicinity of the anastomosis, thereby increasing the patency of the graft.

Preferably the non-circular periphery comprises at least one intraluminal protrusion such as a helicoidal protrusion. This structure entrains the fluid flow to impart rotational flow velocity components.

Preferably the period and/or height of the or each protrusion is not constant. This enables there to be a smooth transition from axial to rotational flow and is more physiologically acceptable.

Preferably the maximum height of the or each protrusion is at least 0.5 mm or at least one tenth of the diameter of the lumen in order to impart significant rotational flow velocity components, such as a maximum of at least 10% of the axial flow velocity.

Preferably the maximum height of the or each protrusion is at least one quarter of the diameter of the lumen, thereby enabling the maximum rotational flow velocity component to be around 25% or more of the mean axial velocity, and to cause the rotational flow field to cover a large area of the cross-section of the lumen.

Preferably there are a plurality of intraluminal protrusions, such as three, spaced at substantially equal angles around the periphery of the lumen to achieve more effective rotational flow induction.

According to another embodiment, preferably the periphery of the lumen is substantially elliptical, oval or other flattened-circular shape. By rotating the orientation of this non-circular periphery along the length of at least a portion of the graft, rotational flow velocity components can be imparted, but whilst having a smooth-walled lumen, without protrusions, which is easier to manufacture and is physiologically a more acceptable conduit shape. However, intraluminal protrusions could also be used in addition to an elliptical or similar shaped lumen.

Preferably the ratio of major diameter to the minor diameter of the elliptical or similar periphery (in other words the aspect ratio) is in the range of from approximately 3:1 to 4:3, such as approximately 3:2. If the ratio is significantly smaller than 4:3, the periphery approaches being circular and is therefore less effective in imparting rotational flow velocity components. If the ratio is significantly greater than 3:1, then the lumen becomes significantly flattened which reduces the cross-sectional area and increases the perimeter of the lumen which reduces the patency of the graft and increases viscous flow losses to the graft which increase the pressure drop along the length of the graft which is undesirable.

Preferably the rotational flow velocity imparting portion of the graft is at the out-flow end of the graft. This enables the in-flow or proximal end of the graft to be a simple conduit for predominantly axial flow for greater throughput whilst the rotational flow is induced at the outflow end where the most profound turbulence and stenosis problems have previously occurred.

Alternatively the rotational flow velocity imparting feature of the graft can extend along substantially its entire length which is simpler to manufacture.

Preferably the maximum imparted rotational flow velocity component is at least 5% and more preferably at least 20% of the mean axial velocity in order to stabilise the flow and alleviate the above mentioned problems.

The invention also provides the method of making a graft as defined in the above aspects of the invention, by the step of extruding a surgically acceptable material through a die whilst rotating the die to form the lumen of non-circular periphery which rotates along the length of at least a portion of the graft.

The invention also provides another method of making a graft as defined in the above aspects of the invention, comprising the steps of: providing a mandrel, whose exterior surface corresponds to the desired interior surface of the graft or portion of the graft; coating the mandrel with a surgically acceptable material; and unscrewing the mandrel to leave the graft or portion thereof.

Thus simple methods of manufacturing this advantageous graft are provided.

The invention also provides a method of treatment and a use of the graft of the invention by implanting the graft in a patient, such as a human.

Embodiments of the invention will now be described, by way of example only, with reference to the accompanying drawings, in which:
Fig. 1 is a schematic cross-sectional view of stenosis at an end-to-side anastomosis;
Figs. 2(a), (b) and (c) illustrate a portion of a graft according to one embodiment of the invention; (a) shows a sequence of transverse cross-sections of the graft; (b) is an end-on view of a transverse cross-section of the graft; and (c) is a perspective view of the graft;
Figs. 3(a) and (b) are plots of the in-plane (rotational) flow velocity components in transverse cross-sections of grafts according to two further embodiments of the invention;
Fig. 4 is a perspective view of a portion of graft according to another embodiment of the invention; and
Fig. 5 is a plot of the in-plane (rotational) flow velocity components in a transverse cross-section of the graft according to the embodiment of Fig. 4.

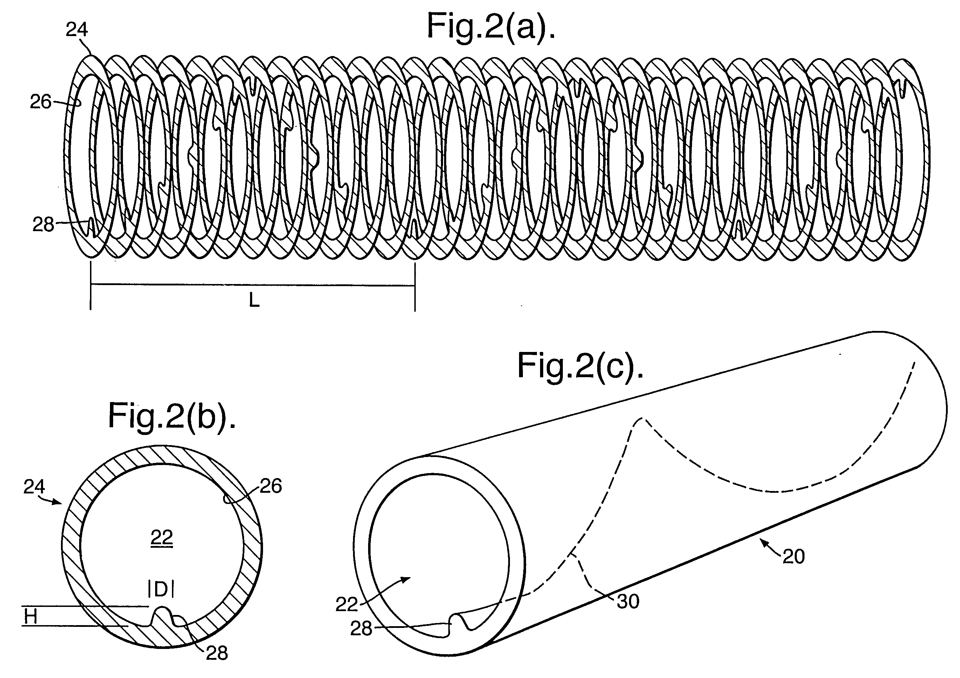

A first embodiment of the invention will be described with reference to Fig. 2. Fig. 2(c) shows a portion of a graft 20 which basically comprises a cylindrical tube having a lumen 22. The graft 20 in this and subsequent embodiments may be an artificial vessel, for example, made of PTFE or other synthetic material, such as polyesters (e.g. Dacron ™), polyurethanes (e.g. ester and carbonate urethanes) and silicones. Fig. 2(a) shows a series of successive transverse cross-sections 24 through the graft 20, only the first one has been labelled 24 and is shown end on in Figure 2(b). The lumen 22 has a periphery 26 which in this example is predominantly circular, but there is an intraluminal protrusion 28 such that overall the periphery of the lumen in transverse cross-section is non-circular. In this embodiment, as shown in Figure 2(b), the height H and the width D of the protrusion 28 are approximately equal to each other and the height H is about one eighth of the internal diameter of the lumen 22. The grafts of this and the following embodiments of the invention typically have a diameter in the range of 5 to 8 mm.

As can be seen in Figure 2(a), the position of the protrusion 28 rotates as a function of position along the length of the graft about an axis parallel to the length of the graft. The locus of the protrusion 28 is indicated by the dashed line 30 in Figure 2(c). Overall the protrusion takes the form of a helicoidal rib in the lumen 22 of the graft 20. The rib has a spatial period L as shown in Figure 2(a).

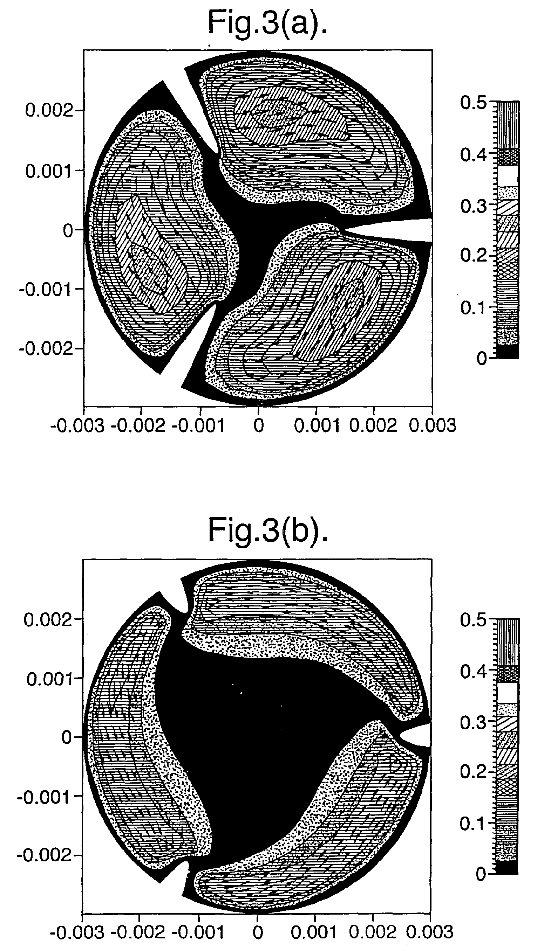

Two further embodiments of the graft of the invention with intraluminal projections are illustrated with reference to Figures 3(a) and 3(b). Each of these figures shows the lumen of the graft taken at a transverse cross-section. As can be seen, the periphery of each lumen is basically circular, but with three intraluminal projections circumferentially spaced at equal angles such that overall the periphery of the lumen is non-circular. Each of the intraluminal protrusions is in the form of a helicoidal rib as described with reference to Figure 2. In the two specific examples of Figures 3(a) and 3(b), the full internal diameter of the lumen is approximately 6 mm and each of the ribs is about one third of a millimetre wide at its base where it joins the inner wall of the graft from which it projects radially inwards.

The difference between the grafts of Figures 3(a) and 3(b) is that in Figure 3(a) the intraluminal protrusions are approximately one quarter of the diameter of the lumen, whereas in Figure 3(b) each protrusion is approximately one tenth of the diameter.

Figures 3(a) and 3(b) illustrate the in-plane fluid velocity components as a function of position in the cross-section of the lumen according to simulation calculations. The in-plane velocity components relate to the rotational flow field. The velocities have been normalised with respect to the mean axial velocity such that the figures in the bar key are the magnitude of the rotational flow velocity as a fraction of the mean axial flow velocity. The arrows give the magnitude and direction of the in-plane velocity at particular points and the contours and shading give the magnitude of the in-plane velocity components.

As can be seen in Figure 3(a), the helical protrusions can entrain a significant rotational flow field with in-plane rotational flow velocity components up to 25% of the average axial velocity. As can be seen in Figure 3(b), with the shorter protrusion, the in-plane rotational flow velocity components are concentrated only in small portions of the flow field near the periphery of the lumen and are up to about 10% of the average axial velocity.

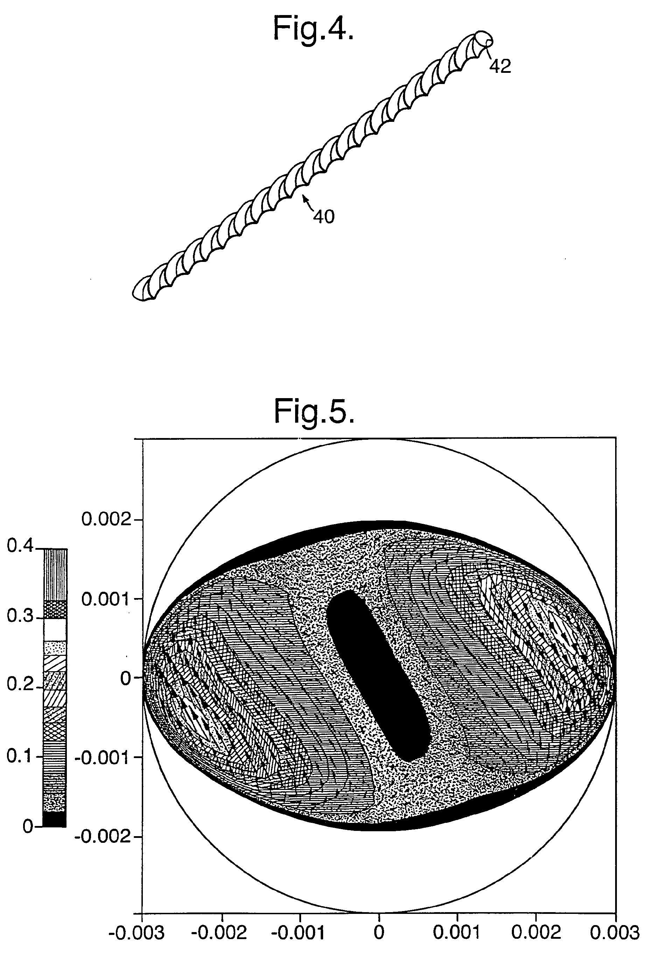

Figure 4 shows a length of graft 40 according to a further embodiment of the invention. In transverse cross-section the graft has a lumen which has an elliptical periphery 42. The orientation of the elliptical periphery rotates as a function of position along the axis of graft such that both the interior and the exterior of the graft in this example have the form of a double screw thread.

Figure 5 is a cross-section showing the in-plane flow velocity components for this embodiment in the same way as in Figure 3. The central ellipse is the lumen of the graft in cross-section. The internal dimension of the lumen across the major axis of the ellipse in this example is about 6 mm, but is only about 4 mm in the perpendicular direction, that is parallel to the minor axis. The aspect ration is thus 3:2. The large circle in Figure 5 represents the area swept out by the ellipse as it rotates along the length of the graft when viewed end on.

As can be seen from the contours and shading in Figure 5, the in-plane velocity components in this example can exceed 25% of the average axial velocity and are significant over large portions of the cross-section of the lumen.

The grafts described above have non-circular rotating internal profiles (protrusions or elliptical shape) for imparting rotational flow, along their entire length. However, other embodiments may have such rotational flow inducing portions along only a part of their length. For example, it is envisaged that the rotational flow imparting portion may be located only along the distal or outflow portion of the graft, because the most profound stenosis problems generally occur at the distal anastomosis.

In preferred embodiments of the graft, the typical spatial period L of rotation of the non-circular cross-section is approximately 3 to 10 times the diameter of the lumen, and in figures the period has been shown as constant. However, it is contemplated that the period need not be uniform along the whole length. Similarly, the height H and width D of any protrusions, or the aspect ration of the elliptical profile, need not be constant, for example, the profile of the lumen could make a gradual transition from circular to non-circular e.g. by gradually increasing the height of the protrusions so that there is a smooth transition from predominantly axial flow to increased rotational flow.

Grafts according to embodiments of the invention can be made, for example, by an extrusion process in which material passes through a die. By rotating the die, the profile of the non-circular lumen forming the graft can be caused to rotate.

Grafts according to the invention can also be made by a process in which a mandrel, whose exterior surface corresponds to the desired interior surface (periphery) of the graft, is coated, e.g. by dipping or spraying, with a surgically acceptable material and then the mandrel is simply unscrewed to leave the graft or at least a desired portion of the graft. The rotation of helicoidal protrusions or the elliptical cross-section enables the mandrel to be conveniently "unscrewed" in this way.

The shape of the exterior of the graft does not have to reflect the interior (as shown in Fig. 4), and the exterior could, for example, be a plain cylinder.

Grafts according to the invention can be used as vessels in human or animal subjects by use of appropriate surgical techniques to implant them.

Whilst specific embodiments of the invention have been described above, it will be appreciated that the invention may be practised otherwise than as described.

## Claims

1. A graft comprising a tubular vessel having a lumen, wherein at least a portion of said lumen, in cross-section, has a non-circular periphery and wherein the orientation of said non-circular periphery rotates as a function of position along the length of said portion, for imparting rotational flow velocity components to a fluid passing along said graft.

2. A graft according to claim 1, wherein said non-circular periphery comprises at least one intraluminal protrusion.

3. A graft according to claim 2, wherein the or each protrusion is helicoidal.

4. A graft according to claim 3, wherein the period of the or each helicoidal protrusion is not constant.

5. A graft according to claim 2, 3 or 4, wherein the height of the or each protrusion is not constant along the length of the lumen.

6. A graft according to any one of claims 2 to 5, wherein the maximum height of the or each protrusion is at least 0.5 mm.

7. A graft according to any one of claims 2 to 6, wherein the maximum height of the or each protrusion is at least one tenth of the diameter of the lumen.

8. A graft according to claim 7, wherein the maximum height of the or each protrusion is at least one quarter of the diameter of the lumen.

9. A graft according to any one of claims 2 to 8, comprising a plurality of said intraluminal protrusions spaced at substantially equal angles around the periphery of said lumen.

10. A graft according to any one of the preceding claims, wherein said periphery of said lumen is substantially elliptical, oval or flattened-circular.

11. A graft according to claim 10, wherein the ratio of the major diameter to the minor diameter of said periphery is in the range of from approximately 3:1 to 4:3, preferably approximately 3:2.

12. A graft according to any one of the preceding claims, wherein said portion of said lumen is at the out-flow end of said graft.

13. A graft according to any one of claims 1 to 11, wherein said portion of said lumen comprises substantially the entire length of said graft.

14. A graft according to any one of the preceding claims for imparting significant rotational velocity flow components to a fluid passing along said graft.

15. A graft according to claim 14, wherein the maximum imparted rotational flow velocity component is at least 5% of the mean axial velocity.

16. A graft according to claim 15, wherein the maximum imparted rotational flow velocity component is at least 20% of the mean axial velocity.

17. A graft according to any one of the preceding claims, wherein the period of rotation of the non-circular periphery is in the range of from approximately 3 to 10 times the diameter of the lumen.

18. A method of making a graft defined in any one of the preceding claims, comprising the step of extruding a surgically acceptable material through a die whilst rotating the die to form said lumen of non-circular periphery which rotates along the length of at least a portion of said graft.

19. A method of making a graft defined in any one of claims 1 to 17, comprising the steps of: providing a mandrel, whose exterior surface corresponds to the desired interior surface of the graft or portion of the graft; coating the mandrel with a surgically acceptable material; and unscrewing the mandrel to leave the graft or portion thereof.

20. Use of a graft according to any one claims 1 to 17 in the human vasculature.

21. A method of treatment comprising the step of implanting a vascular graft according to any one of claims 1 to 17 in a patient.

22. A method of alleviating stenosis in a living vessel as a result of joining a vascular graft to said vessel, said method comprising the step of implanting said graft wherein said graft comprises at least a portion for imparting rotational flow velocity components to a fluid passing along said graft.

23. A method according to claim 22 wherein said living vessel is a blood vessel and said fluid is blood.
